# EUROPEAN PATENT SPECIFICATION

(11) **EP 2 477 661 B1**
(45) Date of publication and mention of the grant of the patent: **14.06.2017**
(21) Application number: 10757186.1
(22) Date of filing: 15.09.2010
(51) Int. Cl.: A61K 51/10

(54) **SELECTIVE DETECTION OF BONE METASTASES IN RENAL CLEAR CELL CARCINOMA**
SELEKTIVER NACHWEIS VON KNOCHENMETASTASEN BEI KLARZELLIGEM NIERENKARZINOM
DÉTECTION SÉLECTIVE DE MÉTASTASES OSSEUSES DANS UN HYPERNÉPHROME À CELLULES CLAIRES

(30) Priority: 15.09.2009 US 242556 P
(43) Date of publication of application: 25.07.2012
(73) Proprietor: Wilex AG, 81675 München (DE)
(72) Inventor: BARTZ, Roman, 81675 München (DE); UZZO, Robert, Ambler Pennsylvania 19002 (US); CHEN, David, 81675 München (DE)
(74) Representative: Weickmann & Weickmann PartmbB
(86) International application number: PCT/EP2010/063530
(87) International publication number: WO 2011/032973

(56) References cited:
- DIVGI CR, PANDIT-TASKAR N, JUNGBLUTH AA, REUTER VE, GÖNEN M, RUAN S, PIERRE C,NAGEL A,PRYMA DA, HUMM J, LARSON SM, OLD LJ, RUSSO P: "Preoperative characterisation of clear-cell carcinoma using iodine-124-labelled antibody chimeric G250 (124I-cG250) and PET in patients with renal masses: a phase I trial", THE LANCET ONCOLOGY, vol. 8, 7 March 2007 (2007-03-07), pages 304-310, XP002615898,
- WU H.,YEN R., SHEN Y., KAO C., LIN C., LEE C.: "Comparing whole body 18F-2-deoxyglucose positron emission tomography and technetium-99m methylene diphosphate bone scan to detect bone metastases in patients with renal cell carcinomas - a preliminary report", JOURNAL OF CANCER RESEARCH AND CLINICAL ONCOLOGY, vol. 128, no. 9, 13 August 2002 (2002-08-13), pages 503-506, XP002615899,
- STEFFENS MG, BOERMAN OC, DE MULDER PH ET AL: "Phase I Radioimmunotherapy of Metastatic Renal Cell Carcinoma with 131I-labeled Chimeric Monoclonal Antibody G250", CLINICAL CANCER RESEARCH, vol. 5, 1 October 1999 (1999-10-01), pages 3268S-3274S, XP002615900,
- DATABASE MEDLINE [Online] US NATIONAL LIBRARY OF MEDICINE (NLM), BETHESDA, MD, US March 2002 BROUWERS A H ET AL: '131 I-cG250 monoclonal antibody immunoscintigraphy versus [18 F]FDG-PET imaging in patients with metastatic renal cell carcinoma: a comparative study.' Database accession no. NLM11891480 & BROUWERS A H ET AL: "131 I-cG250 monoclonal antibody immunoscintigraphy versus [18 F]FDG-PET imaging in patients with metastatic renal cell carcinoma: a comparative study.", NUCLEAR MEDICINE COMMUNICATIONS MAR 2002, vol. 23, no. 3, March 2002 (2002-03), pages 229-236, ISSN: 0143-3636
- GROVES ET AL: "Non-[<18>F]FDG PET in clinical oncology", LANCET ONCOLOGY, LANCET PUBLISHING GROUP, LONDON, GB, vol. 8, no. 9, 1 September 2007 (2007-09-01), pages 822-830, XP022217815, ISSN: 1470-2045, DOI: 10.1016/S1470-2045(07)70274-7
- BOUCHELOUCHE K ET AL: "Recent developments in urologic oncology: positron emission tomography molecular imaging", CURRENT OPINION IN ONCOLOGY, CURRENT SCIENCE LTD, US, vol. 20, no. 3, 1 May 2008 (2008-05-01), pages 321-326, XP009185845, ISSN: 1040-8746

## Description

The present invention refers to the detection of bone metastases in renal cell carcinoma (RCC) and suitable reagents therefor.

Renal cell carcinoma (RCC) is the most common type of kidney cancer in the kidneys, accounting for 80% to 90% of cases. The incidence of renal cell cancer has been rising steadily. Approximately 57,760 new cases of renal cell carcinoma (RCC) are estimated to occur in 2009 in the United States, with an expected mortality of 12,980 (CA Cancer J Clin 2009; 59:225-249).

The initial diagnosis of RCC is usually made with ultrasound, CT, or MRI. Most cases (50-70%) are discovered as incidental renal masses during the course of a cross-sectional imaging procedure obtained for other purposes. At present, surgical resection of the renal tumor is the recognized treatment of choice in this situation. On presentation, nearly 20% of renal masses are stage IV with an additional 20% deemed "locally advanced" by current imaging modalities.

A renal mass suspicious for RCC is ultimately found to be benign in approximately 20% of cases. Of malignant renal tumors approximately 15-20% of cases represent an indolent or less aggressive type, most typically non-clear cell histologies such as chromophobe or papillary, while approximately 75-85% of cases represent a clear cell type of renal cell carcinoma (ccRCC). Clear cell renal cell carcinoma is an aggressive phenotype and is generally associated with a worse clinical prognosis than other types of RCC. Around 90% of patients who present with, or later develop, metastases from renal cancer have clear-cell carcinoma.

The prognosis and treatment for metastatic RCC is significantly worse (approx. 12-24 months) than for localized disease amenable to complete surgical resection. Therefore, it is important to identify distant spread as early as possible to provide the optimal treatment for this stage of RCC.

G250 is a monoclonal antibody that has been shown in animal and human studies to bind to clear-cell renal cell carcinoma. The antigen target of G250 is an epitope of carbonic anhydrase IX. By immunohistology, carbonic anhydrase IX (CA-IX) is found in more than 94% of human clear-cell renal carcinomas. A chimeric form of the antibody (designated cG250) has been generated to be less immunogenic and applicable for human diagnostic or therapeutic clinical applications. An intriguing feature of this antibody is its exceptionally avid targeting in renal cell carcinomas, with biopsy-based studies showing tumor uptake approaching 0-1 %/g, the highest recorded avidity for any solid tumor. G250, in its murine and chimeric forms, has been used extensively as a potential therapeutic agent, either alone or conjugated with a radioactive isotope.

The targeting and affinity of G250 to clear cell renal carcinoma has spurred interest in its development as a diagnostic marker, to predict a *priori* this usually aggressive phenotype.

Recently, it has been reported that PET imaging was conducted using ¹²⁴I-labeled cG250. A total of 26 patients with renal masses examined using ¹²⁴I-cG250 PET/CT-imaging prior to surgery. The sensitivity of ¹²⁴I-cG250 PET for clear cell kidney carcinoma in this trial was 94%; the negative predictive value was 90%, and specificity and positive predictive value were both 100%. No drug-related immediate or delayed adverse events were reported in this trial (Divgi et al., Lancet Oncol. 8 (2007) 304-310). Further, the study describes the visualization of a lung metastasis and a lymph node metastasis in two patients. The detection of bone metastases is not described.

However, the detection of bone metastases is of high importance, because a high number of RCC patients already have metastasis when the disease is noticed and/or diagnosed (approx. 30%), of which approximately 22-32% of these represent bone metastases.

Brouwers et al. (Nuclear Medicine Communications, 23, 2002, 229-236) disclose a comparison between ¹³¹I-cG250 and [18]-fluorodeoxyglucose ([18F]FDG) in the detection of metastatic renal-cell carcinoma, showing that [18F]FDG-PET is superior to an antibody approach using a labelled cG250. The authors found that only 30% of metastatic lesions were visualized by ¹³¹I-cG250, while nearly 70% were detected by [18F]FDG.

At present, so called nuclear bone scans are used to diagnose or help to diagnose a number of conditions relating to bones. A bone scan is a nuclear scanning method, wherein the patient is injected with a small amount of radioactive material such as e.g. Technetium-99m and then scanned with a device sensitive to the radiation emitted by the injected material. The more active the bone turnover, the more radioactivity is localized by the bones. Some tumors or metastases show up as areas of increased uptake due to bone growth or breakdown. However, not all tumors are easily seen on the bone scan. Further, benign conditions such as old fractures or other osseous injuries, arthritis and infections may show up as "positive" on the bone scan requiring further evaluation to distinguish from true malignant involvement by kidney cancer cells. Thus, the specificity of this method for detecting bone metastases is suboptimal. Needless to say, this method represents additional radiation exposure, cost and anxiety for the patient.

Early and specific detection of bone metastases allows for suitable therapeutic counter-measures such as surgical resection, focal radiotherapy, or systemic medical treatment. Moreover, confirmed bony involvement may completely alter the therapeutic strategy by making surgery unnecessary or inappropriate.

Thus, there is a high need to provide a sensitive and reliable method of detecting bone metastases in RCC.

The present inventors have found that ¹²⁴I-labelled antibodies are a useful and very specific reagent for detecting bone metastases.

Thus, one aspect of the present invention refers to a method for detecting bone metastases in renal cell carcinoma (RCC), particularly in clear cell renal carcinoma (ccRCC), comprising administering via a pre-laid infusion an effective amount of an ¹²⁴I-labelled antibody directed against CA-IX or an antigen-binding fragment thereof to a subject and determining radiation from said subject, wherein the determination of radiation comprises Positron Emission Tomography (PET) imaging.

Further, the present application describes an ¹²⁴I-labelled antibody or an antigen-binding fragment thereof directed against anhydrase IX for use in detecting bone metastases in renal cell carcinoma (RCC), particularly in clear cell renal carcinoma (ccRCC).

According to the present invention an ¹²⁴I-labelled antibody or an antigen-binding fragment thereof is used. This antibody or antibody fragment is directed against CA-IX, particularly human CA-IX. Antibodies against carbonic anhydrase IX and the manufacture of such antibodies is e.g. described in WO 93/18152.

In a particularly preferred embodiment the antibody is a monoclonal G250 antibody, particularly a chimeric G250 antibody or an antigen-binding fragment thereof. The G250 antibody, its antigen-binding sites and a hybridoma cell capable of producing this antibody, have been described in WO 02/062972. A hybridoma cell capable of producing the G250 antibody was deposited under the Budapest Treaty for the Deposit of Microorganisms on September 11, 2001 at Deutsche Sammlung von Mikroorganismen und Zellkulturen GmbH (DSMZ), Mascheroder Weg 1 b, 38124 Braunschweig, Germany under the Accession Number DSM ACC 2526.

According to the invention the term "G250 antibody" covers any antibody including multispecific antibodies (e.g. bispecific antibodies) and antibody fragments as long as they exhibit the desired activity, i.e. at least one G250 antigen-binding site. The antibody may be an IgM, IgG (e.g. IgG₁, lgG₂, IgG₃ or IgG₄) IgD, IgA or IgE, particularly IgG antibody, a recombinant antibody or an antibody fragment obtained by proteolytic methods or by recombinant DNA methods.

The term "monoclonal antibody" as used herein refers to an antibody obtained from a population of substantially homogeneous antibodies, i.e., the individual antibodies comprising the population are identical, except for possibly naturally occurring mutations that may be present in minor amounts.

The term "antibody" as used herein refers to any polypeptide containing at least one G250 antigen-binding site, i.e. at least the CDR3 region of the G250 heavy chain and/or the CDR3 region of the G250 light chain or a variant G250 CDR3 region having an identity of at least 80%, preferably at least 90% to the original G250 CDR3 region on the amino acid level, provided that the variant CDR3 region has equivalent antigen-binding characteristics, particularly affinity and specificity compared to the original CDR3 region. The G250 CDR3 regions are disclosed in WO 02/062972.

Preferably, the term "antibody" herein includes chimeric antibodies, humanized and fully humanized antibodies, single chain antibodies, e.g. sFv antibody fragments, diabody fragments, proteolytic or recombinant antibody fragments such as Fv-, Fab-, Fab'- or F(ab')₂-fragments or other antigen-binding subsequences of antibodies. The antibody may also be a fusion or a conjugate with other entities.

The antibodies herein specifically include chimeric antibodies in which a portion of the heavy and/or light chain including the antigen-binding site is identical with or homologous to corresponding sequences derived from the original hybridoma cell line G250, while the remainder of the chains is identical with or homologous to corresponding sequences derived from other species or belonging to another antibody class or subclass as well as fragments of such antibodies as long as they exhibit the desired biological activity. More preferably, the chimeric antibody comprises variable regions, e.g. the complement-determining regions (CDRs) and the framework regions from the heavy chain and the light chain of the original G250 monoclonal antibody and constant human sequences, particularly constant human kappa light chain and gamma heavy chain sequences. The manufacture of chimeric antibodies is described e.g. by Morrison et al. (Proc. Natl. Acad. Sci. USA 81 (1984), 6851-6855).

Further, antibody herein specifically includes humanized antibodies or fully human antibodies. Humanized antibodies are immunoglobulins, immunoglobulin chains or fragments thereof which contain minimal sequence derived from non-human immunoglobulin. More particularly, humanized antibodies are human immunoglobulins in which residues from a CDR of a given human antibody are replaced by residues from the G250 CDR, particularly the CDR1, 2 and/or 3 region of the heavy and/or light chain. Furthermore, humanized antibodies may comprise residues which are found neither in the recipient human antibody, nor in the imported G250 CDR sequences. These modifications are made to further refine and optimize antibody performance. In general, the humanized or fully human antibody will comprise substantially all of at least 1, and typically 2, variable domains, in which all or substantially all of the CDR regions correspond to those of the original G250 immunoglobulin and all or substantially all of the framework regions and constant regions are those of a human immunoglobulin sequence. The manufacture of humanized antibodies is described, e.g. in Jones et al. (Nature 321 (1986), 522-525), Riechmann et al. (Nature 332 (1988), 323-329) and Presta (Curr. Op. Struct. Biol. 2 (1992), 332-339).

Further, antibodies specifically include single-chain antibodies such as single-chain Fv antibody fragments comprising the VH and VL domains of an antibody, wherein these domains are present in a single polypeptide chain. Generally, the Fv polypeptide further comprises a polypeptide linker between the VH and VL domains which enables the sFv to form the desired structure for antigen binding. The manufacture of sFv antibodies is described e.g. by Plückthun in: The Pharmacology of Monoclonal Antibodies, Vol. 113, Rosenburg and Moore, Eds., Springer Verlag, NY, pp. 269-315 (1994), Barbas III (Methods: Companion Methods Enzymol. 2 (1991), 119) and Hoogenboom et al. (Immunol. Rev. 130 (1992), 41-68).

Further, antibodies specifically include diabodies, i.e. small antibody fragments with two antigen-binding sites, which fragments comprise a heavy chain variable domain connected to a light chain variable domain in the same polypeptide chain. By using a linker that is too short to allow pairing between the two domains on the same chain, the domains are forced to pair with the complementary domains of another chain and create two antigen binding sites. The manufacture of diabodies is described e.g. by Hollinger et al., (Proc. Natl. Acad. Sci. USA 90 (1993), 6444-6448.

According to the present invention the antibody is conjugated, e.g. by covalently coupling the radioactive isotope ¹²⁴I. This isotope is a positron emitter that can be attached to antibodies e.g. as described by Larsson et al. (J. Nucl. Med. 33 (1992), 2020-2023) or US 5,185,142.

Preferably the radiolabeling of the antibody is accomplished by covalent iodination, particularly with the lodogen Reagent (1,3,4,6-tetrachloro-3α,6α-diphenyl glycoluril). Iodogen labeling is a solid phase oxidative method that is similar to the Chloramine-T method, but is generally considered to be milder, since the reaction takes place on the surface of the oxidant, minimizing exposure of the substrate (Salacinzki, P.R.P., et al., Anal.Biochem. 117:136 (1981)).

The ¹²⁴I-labelled antibody is particularly useful in human medicine, i.e. for administering to human patients, e.g. human patients suffering from renal masses, in particular RCC, more particularly ccRCC.

In one embodiment of the present invention, the patient is a pre-operative patient with renal masses, e.g. a preoperative patient, who has been diagnosed for RCC or ccRCC and scheduled to undergo surgery. In this embodiment the patient has particularly a high risk for metastases, in particular bone metastases, and is particularly diagnosed for ccRCC.

More preferred the patient is a pre-operative patient with renal masses who has been scheduled for a PET-Scan with ¹²⁴I-labelled cG250 for the preoperative determination of the type of renal cancer, e.g. ccRCC and non-ccRCC. These patients have the additional benefit that they have to undergo only one diagnostic procedure and get both the information of the type of the cancer and the presense of bone metastases.

A further preferred patient is a pre-operative patient with renal masses which has been diagnosed by any other method for ccRCC, e.g. by biopsy and histological analysis of the sample.

In another embodiment of the present invention, the patient is a patient with the diagnosis of renal masses who had undergone surgery, particularly a postoperative RCC or more particularly ccRCC patient, more particularly a patient with a high risk of recurrence. Pathologic staging from surgery can identify patients who have a particularly increased risk of developing RCC recurrence.

Further preferred patients are people who have a history of a resected kidney tumor who present with a bony lesion. In this embodiment the use of the inventive ¹²⁴I-labelled antibodies advantageously provide reliable information if that bony lesion is ccRCC.

In human medicine, the antibody is usually administered in a dose from about 1 mg to about 50 mg, preferably in a dose from about 5 mg to about 20 mg, and more preferably in a dose of about 10 mg. The specific activity of the radiolabelled antibody is preferably about 15 to about 20 MBq/mg, more preferably about 18 to about 19 MBq/mg.

The antibody is usually administered as a pharmaceutical composition with a pharmaceutically acceptable carrier, e.g. physiological saline solution, optionally comprising a protein stabilizer such as human serum albumin (HSA). The antibody is preferably administered by infusion.

In a preferred embodiment, the patient scheduled for ¹²⁴I antibody administration is subjected to a thyroid blockade by administering an iodide salt, e.g. potassium iodide. For this purpose potassium iodide tablets, e.g. IOSAT TM can be used, and/or potassium iodide can be applied orally in liquid form, e.g. as drops such as SSKI^{®}.

The measurement of ¹²⁴I involves Positron Emission Tomography (PET) or PET/CT. The patient is scanned in several bed positions to generate a Scan over the whole body. The PET imaging is performed preferably within 7 ± 2 days of infusion of the ¹²⁴I antibody, in particular 5 ± 2 days after the ¹²⁴I antibody infusion, in order to obtain the optimal imaging results.

Further, the present invention shall be explained in more detail by the following examples.

### Example 1

### Administration of ¹²⁴I-labelled chimeric G250 antibody for the detection of metastases in RCC

### 1. Preparation and Administration of Antibody

The radiolabeled antibody (5 mCi/10mg ¹²⁴I-cG250) in a volume of 10ml 15% HSA was provided in a sterile vial together with sterile normal saline solution. Prior to intravenous infusion, the radiolabeled antibody (10 ml) was diluted in 20 ml of sterile normal saline to make it a 30 ml solution for intravenous infusion.

The product label included the patient code and the calibration date and time (i.e. the date and time when the 5 mCi of radioactivity was present). The product was used within 24 h after the calibration time.

A syringe infusion pump (Graseby 3400 or equivalent) was used for continuous infusion of the 30 ml at a rate of 2 ml/min over 15 minutes on the day of infusion. The intravenous line, including a 3-way stopcock, included an in-line 0.22 micron filter. The infusion was administered after ensuring adequate intravenous access (preferably in an antecubital vein) using a 5% human serum albumin (HSA) solution. At completion of infusion, the syringe and intravenous line were flushed using at least 10 ml of 5% HSA.

The quantity of radioactivity given to the patient was calculated retrospectively based on the calibration date/time compared to the administration date/time using the known decay data ¹²⁴I. The exact time (date/hour/minute) of administration of ¹²⁴I-cG250 to the patient was recorded. Findings regarding the administered radiation dose obtained via the calculation method were analyzed for reproducibility and variability.

In addition, the administered dose was measured for each patient using a suitable device and the results recorded on the Data Transmittal Form, which was completed for each image.

### 2. Ancillary Therapy: Thyroid Block

All patients received a daily oral dose of potassium iodide (SSKI^{®}, Upsher-Smith) to block uptake of free iodine in the thyroid. 600 mg were given 24 hours prior to administration of ¹²⁴I-cG250 on day 1 followed by 600 mg per day for 2 weeks after infusion or until the day of surgery, whichever occurred first.

### 3. ¹²⁴I-cG250 PET/CT Imaging

¹²⁴I PET/CT imaging of the whole body was performed 5±2 days after ¹²⁴I-cG250 infusion. Attenuation-corrected PET/CT images were acquired.

### 4. Results

The classification of the tumors has been carried out according the Guidelines of the International Union Against Cancer (UICC) (TNM Classification of Malignant Tumors, Edited by L.H. Sobin and Ch. Wittekind, Sixth Edition, John Wiley & Sons, New York, NY. 2002)

The PET scans of 3 patients are shown in the figures. The patients show well-detectable bone metastases. Bone metastases are indicated by arrows.

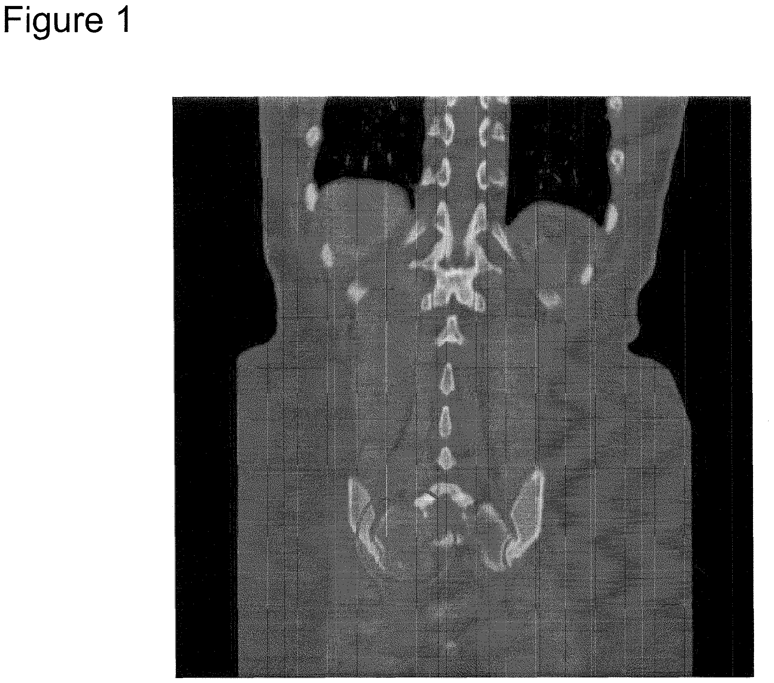

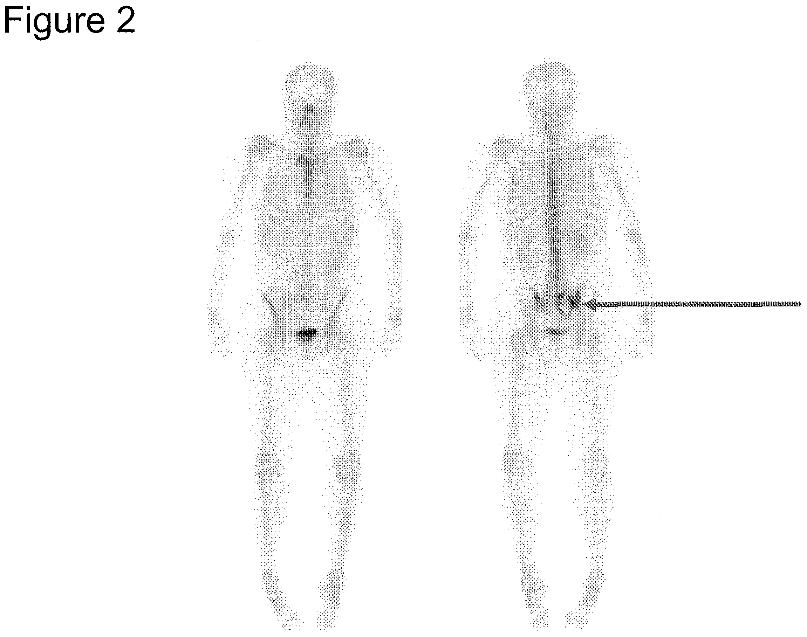

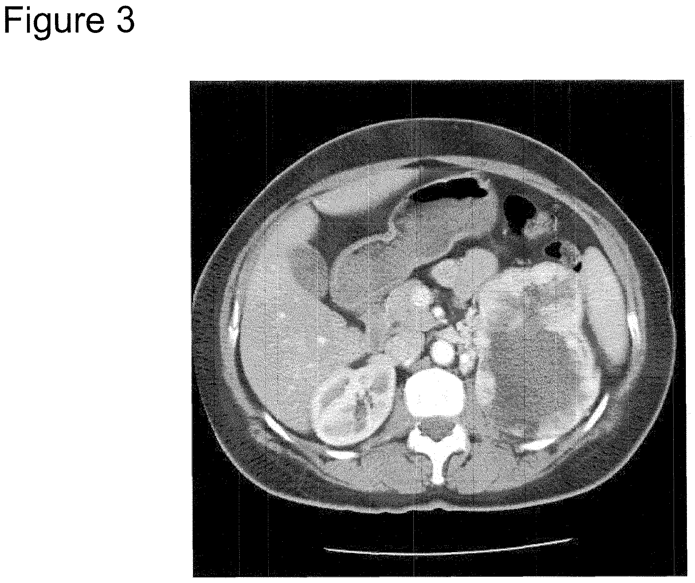

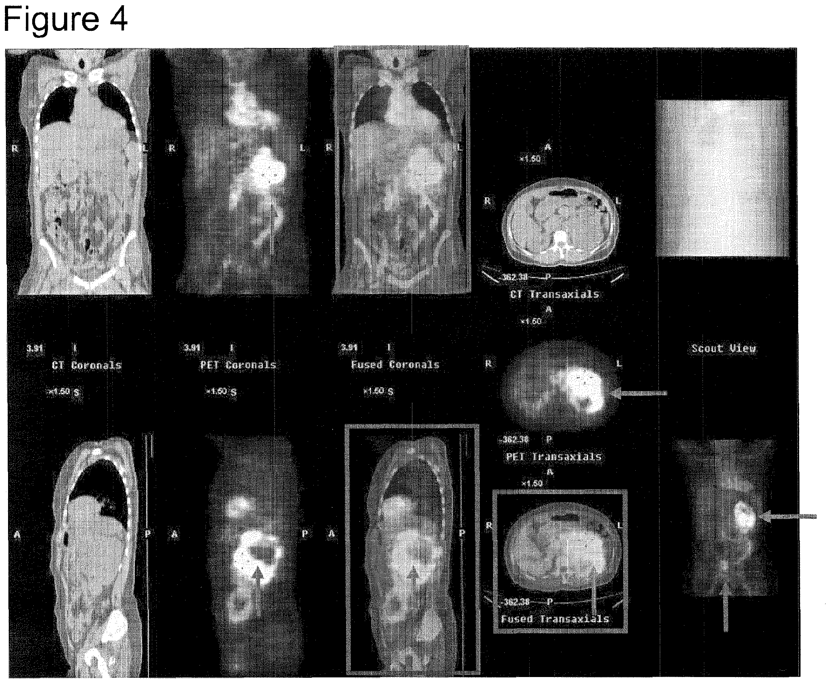

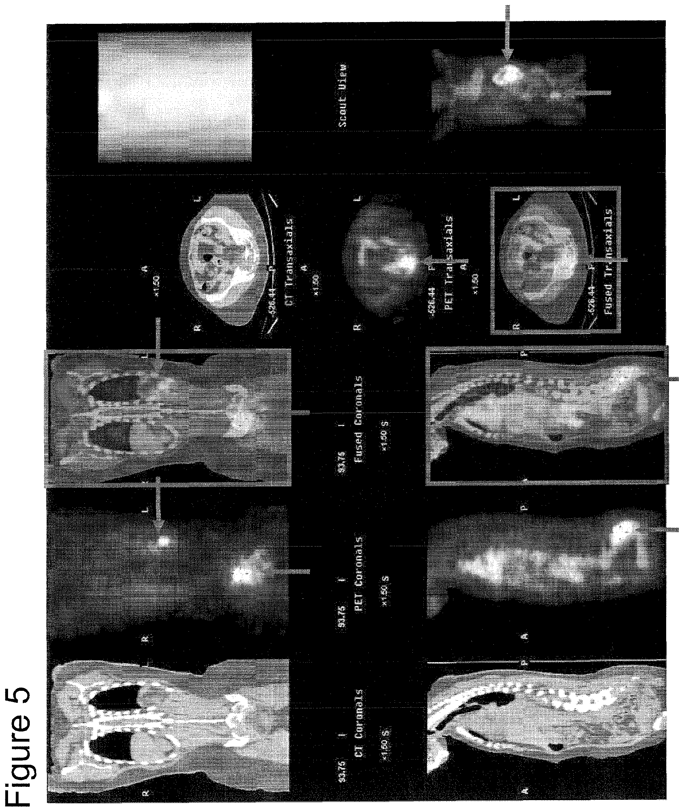

Figures 1-5 show the findings from a 48 years old female patient. The initial patient symptom was pain in the area of the lower back. The inventive method reveals metastatic ccRCC with a bone metastasis in the sacral bone.
Figure 1 shows an initial low-contrast CT scan of the abdomen which reveals a non-specific bony defect in the right sacrum (circled).
Figure 2 shows a bone scan with Tc⁹⁹ indicating a lytic bone metastasis in the sacral bone (indicated by an arrow).
Figure 3 shows a CT scan, where a large renal mass in the left kidney can be identified.
Figures 4 and 5 show localizing CT scans, ¹²⁴I-cG250 PET scans and the corresponding combination PET/CT pictures (framed). The sacrum metastasis and the primary renal tumor are indicated by arrows.
Figures 6-10 show the findings from a 55 years old male patient. The inventive method reveals metastatic ccRCC with bone metastases in the rib, the clavicle, and the scapula.

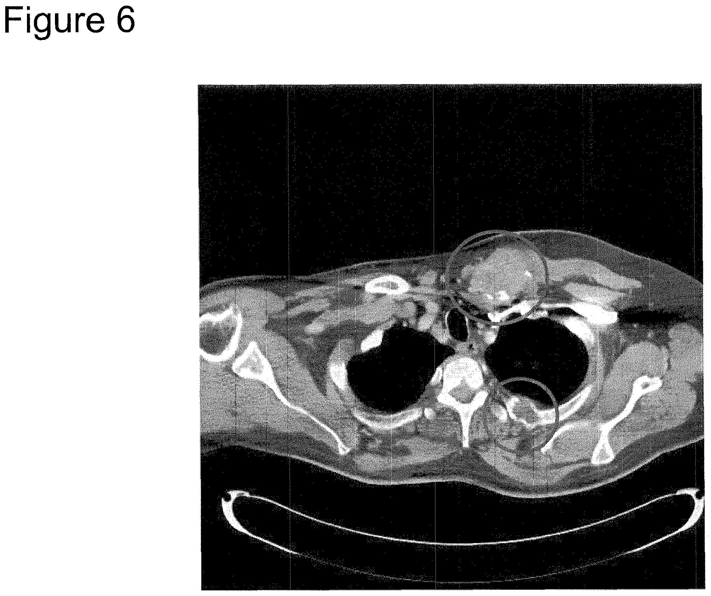

Figure 6 shows an initial CT scan of the chest, with the bony defects seen in the posterior rib and clavicle (circled).

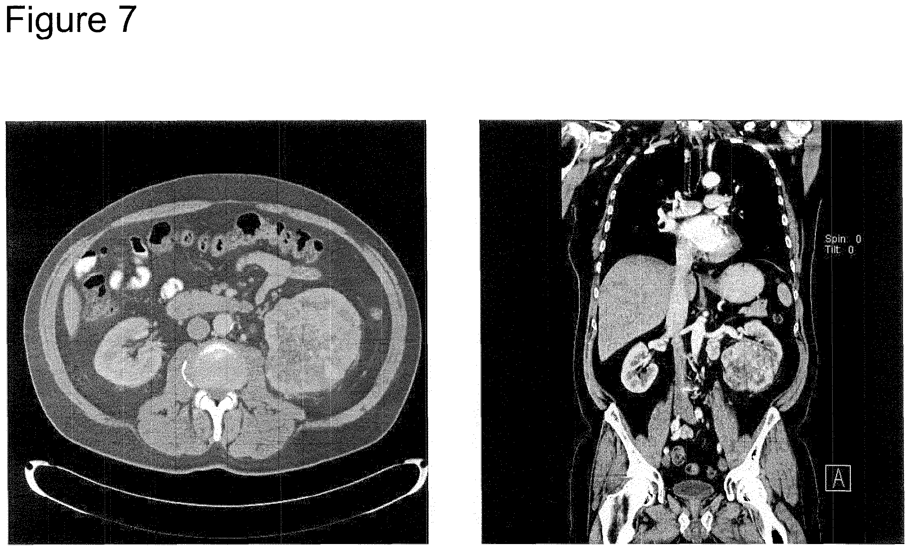

Figure 7 shows CT scans, where a renal mass mostly replacing the left kidney can be identified.

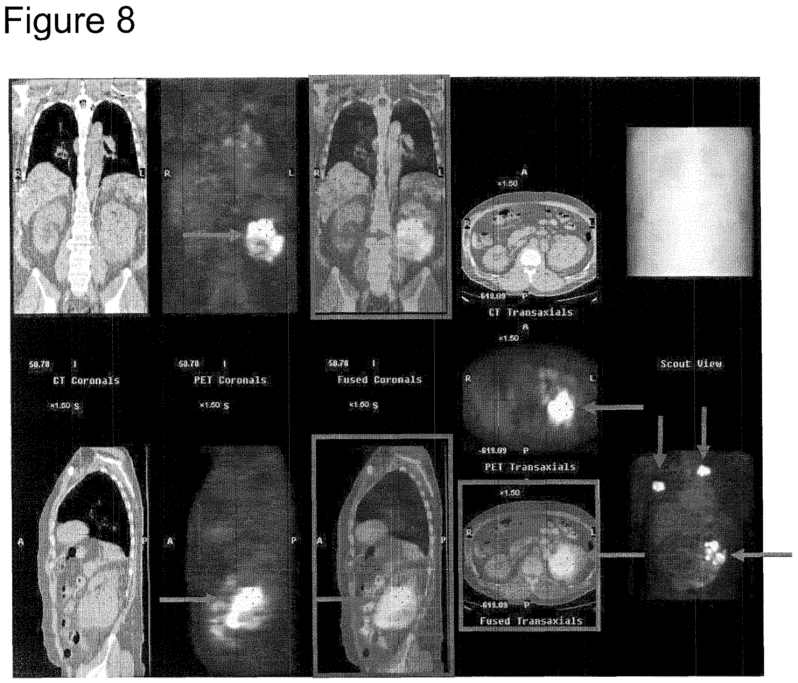

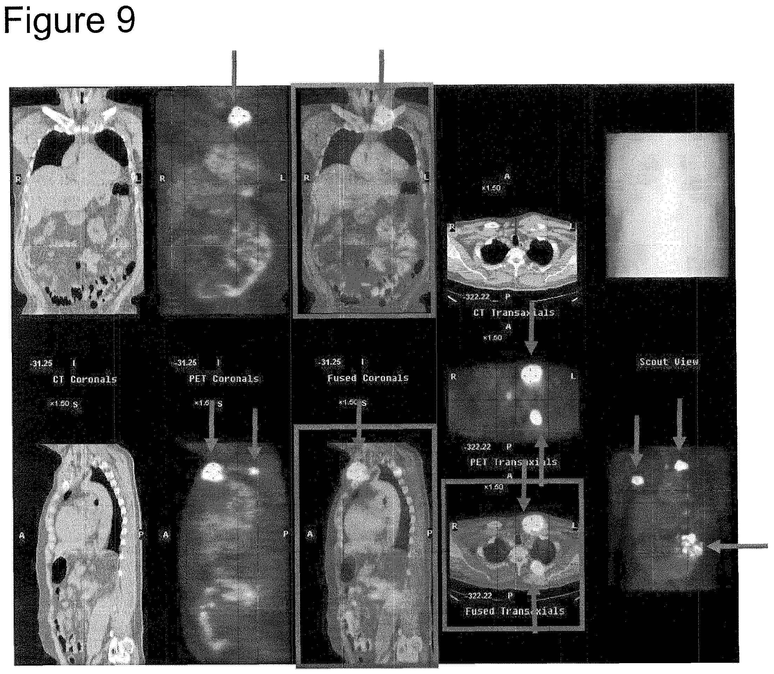

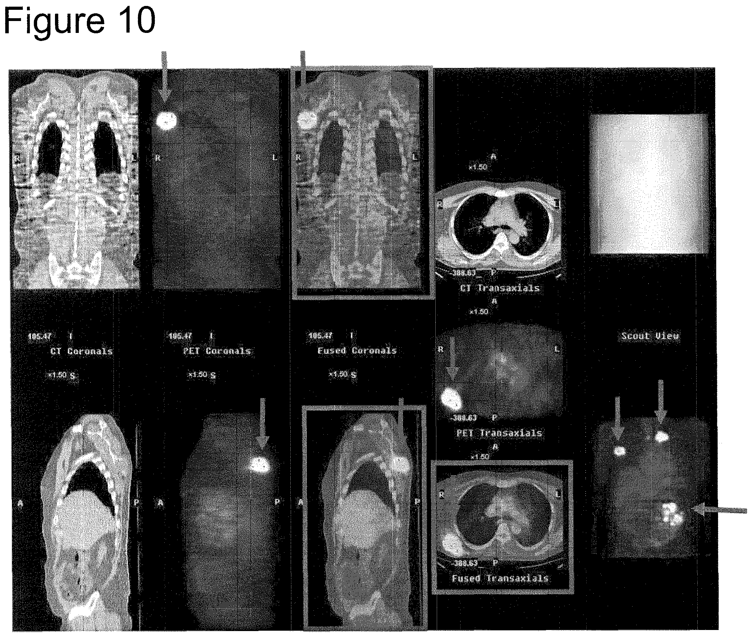

Figures 8-10 show CT scans, ¹²⁴I-cG250 PET scans and the corresponding combination PET/CT pictures (framed in red). A rib metastasis, clavicle metastasis and scapula metastasis and the primary renal tumor are indicated by arrows.

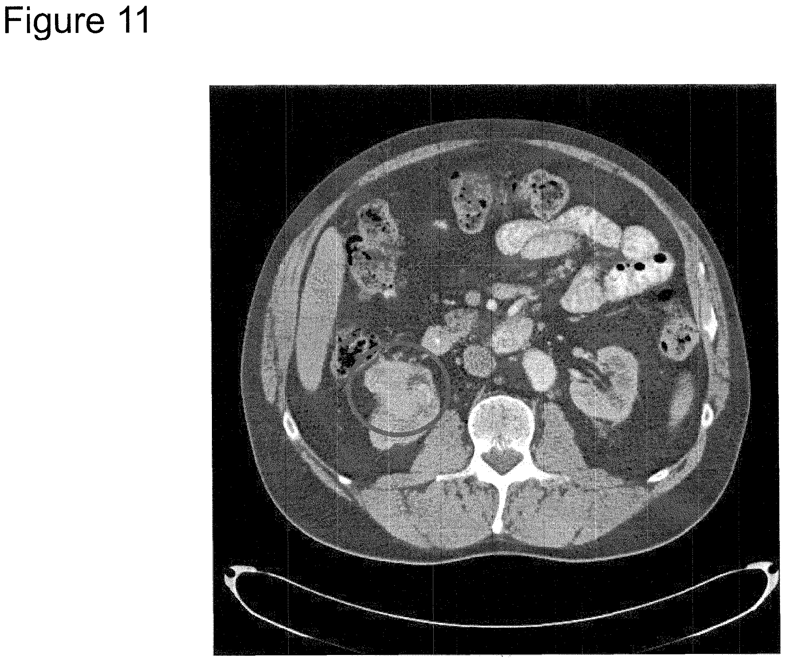

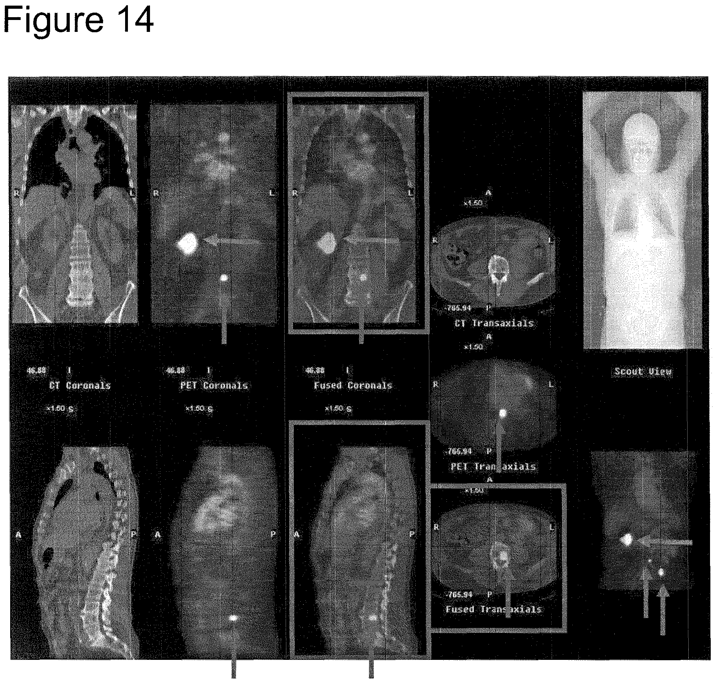

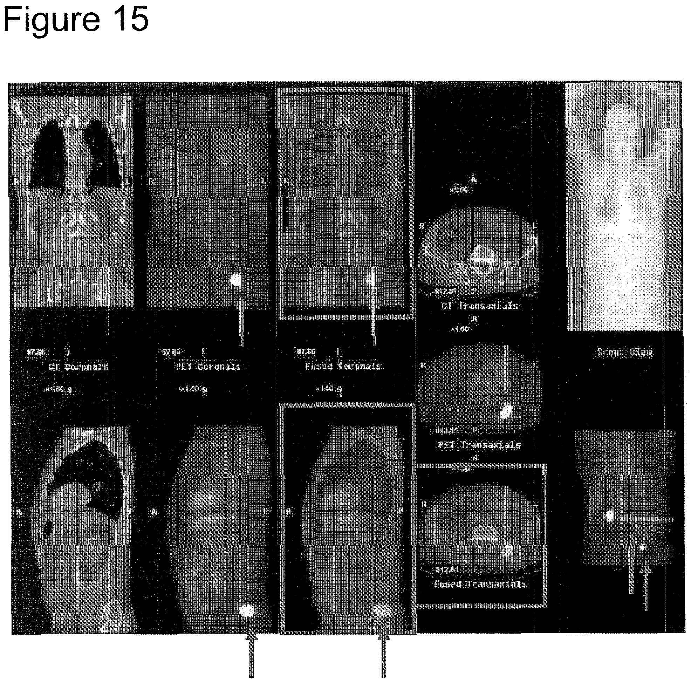

Figures 11-15 show the findings from a 68 years old male patient. The inventive method reveals a metastatic ccRCC disease with bone metastases in the spine and iliac wing.
Figure 11 shows an initial CT scan of the abdomen with the right renal mass identified (circled).

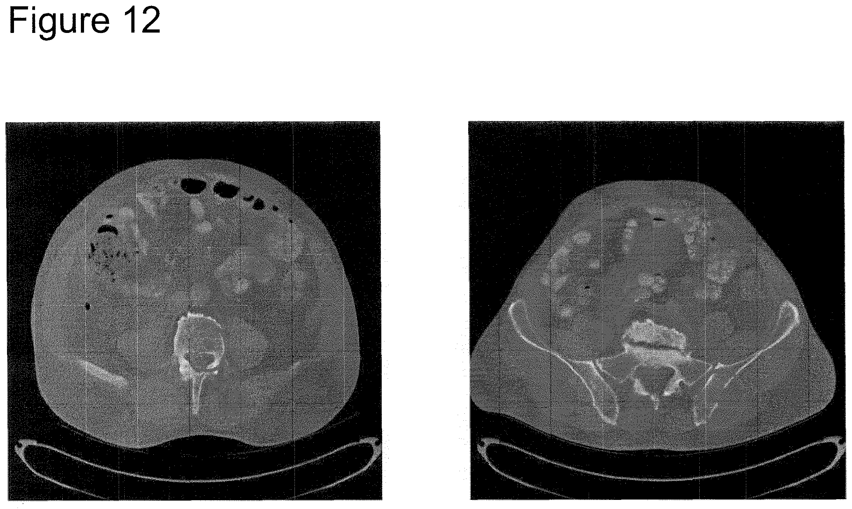

Figure 12 shows a CT scan, where a subtle lytic abnormality in the spine and the iliac wing can be seen (circled).

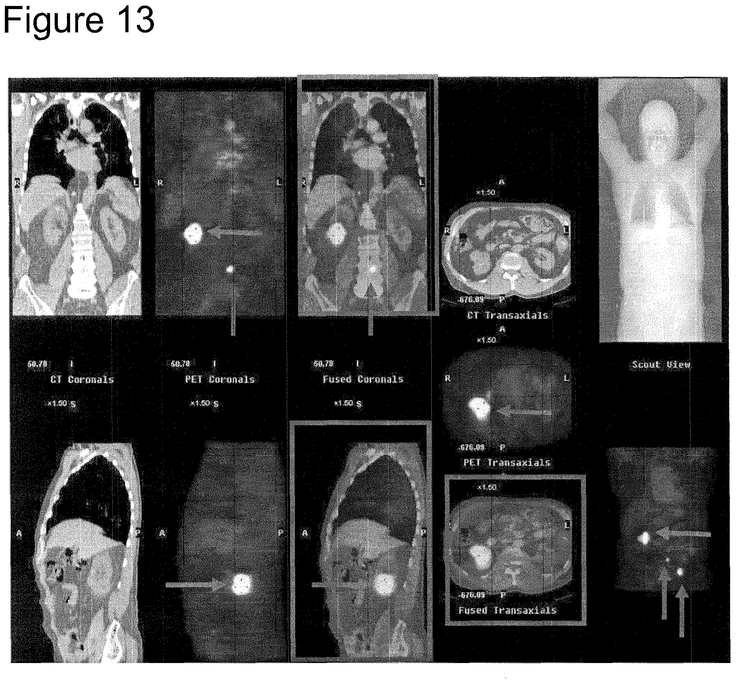

Figures 13-15 show CT scans, ¹²⁴I-cG250 PET scans and the corresponding combination PET/CT pictures (framed). A spine metastasis and a iliac wing metastasis are indicated and the primary renal tumor are indicated by arrows.

## Claims

1. A method for producing an image of bone metastases in renal cell carcinoma (RCC) comprising infusing an effective amount of an ¹²⁴I-labelled antibody directed against carbonic anhydrase IX or an antigen-binding fragment thereof to a subject and determining radiation from said subject, wherein the determination of radiation comprises Positron Emission Tomography (PET) imaging.

2. The method according to claim 1, wherein bone metastases are detected in clear cell renal carcinoma (ccRCC).

3. The method according to claim 1 or 2, wherein the subject is a human patient.

4. The method according to claim 3, wherein the patient is a preoperative patient.

5. The method according to claim 3, wherein the patient is a postoperative patient.

6. The method according to any one of claims 1-5, wherein the antibody is a monoclonal G250 antibody, particularly a chimeric G250 antibody or an antigen-binding fragment thereof.

7. The method according to any one of claims 1-6, wherein the antibody is administered in a dose from 5 mg to 20 mg, particularly in a dose of about 10 mg.

8. The method according to any one of claims 1-7, wherein the activity of the antibody is 15-20 MBq/mg, particularly 18-19 MBq/mg.

## Patentansprüche

1. Verfahren zur Herstellung einer Abbildung von Knochenmetastasen im Nierenzellkarzinom (RCC), umfassend Infundieren einer wirksamen Menge eines ¹²⁴I-markierten Antikörpers, der gegen Carboanhydrase IX gerichtet ist oder eines Antigen-bindenden Fragments davon in ein Individuum, und Bestimmen der Strahlung von dem Individuum, wobei die Bestimmung von Strahlung Positronen-Emissions-Tomographie (PET) -Bildgebung umfasst.

2. Verfahren nach Anspruch 1, wobei Knochenmetastasen im klarzelligen Nierenkarzinom (ccRCC) nachgewiesen werden.

3. Verfahren nach Anspruch 1 oder 2, wobei das Individuum ein menschlicher Patient ist.

4. Verfahren nach Anspruch 3, wobei der Patient ein präoperativer Patient ist.

5. Verfahren nach Anspruch 3, wobei der Patient ein postoperativer Patient ist.

6. Verfahren nach einem der Ansprüche 1-5, wobei der Antikörper ein monoklonaler G250-Antikörper ist, insbesondere ein chimärer G250-Antikörper oder ein Antigen-bindendes Fragment davon.

7. Verfahren nach einem der Ansprüche 1-6, wobei der Antikörper in einer Dosis von 5 mg bis 20 mg verabreicht wird, insbesondere in einer Dosis von etwa 10 mg.

8. Verfahren nach einem der Ansprüche 1-7, wobei die Aktivität des Antikörpers 15-20 MBq/mg beträgt, insbesondere 18-19 MBq/mg.

## Revendications

1. Procédé pour produire une image de métastases osseuses dans le carcinome à cellules rénales (CCR) comprenant l'introduction d'une quantité efficace d'un anticorps marqué avec ¹²⁴I dirigé contre l'anhydrase carbonique IX ou d'un fragment liant l'antigène de celui-ci chez un sujet et la détermination du rayonnement provenant dudit sujet, où la détermination du rayonnement comprend une imagerie par tomographie par émission de positrons (TEP).

2. Procédé selon la revendication 1 où les métastases osseuses sont détectées dans le carcinome à cellules rénales à cellules claires (CCRcc).

3. Procédé selon la revendication 1 ou 2 où le sujet est un patient humain.

4. Procédé selon la revendication 3 où le patient est un patient en phase pré-opératoire.

5. Procédé selon la revendication 3 où le patient est un patient en phase post-opératoire.

6. Procédé selon l'une quelconque des revendications 1-5 où l'anticorps est un anticorps G250 monoclonal, en particulier un anticorps G250 chimérique ou un fragment liant l'antigène de celui-ci.

7. Procédé selon l'une quelconque des revendications 1-6 où l'anticorps est administré en une dose de 5 mg à 20 mg, en particulier en une dose d'environ 10 mg.

8. Procédé selon l'une quelconque des revendications 1-7 où l'activité de l'anticorps est 15-20 MBq/mg, en particulier 18-19 MBq/mg.
